# EUROPEAN PATENT APPLICATION

(11) **EP 4 273 226 A1**
(43) Date of publication of application: **08.11.2023**
(21) Application number: 21914380.7
(22) Date of filing: 28.12.2021
(51) Int. Cl.: C12N 1/21, C12N 15/31, C07K 14/34, C12N 15/77, C12P 13/18, C12R 1/15

(54) **RECOMBINANT STRAIN FOR PRODUCING L-GLUTAMIC ACID BY MODIFYING GENE BBD29_09525, AND CONSTRUCTION METHOD THEREFOR AND APPLICATION THEREOF**

(30) Priority: 30.12.2020 CN 202011631266
(71) Applicant: Inner Mongolia Eppen Biotech Co., Ltd., Chifeng, Inner Mongolia 024000 (CN)
(72) Inventor: JIA, Huiping, Chifeng, Inner Mongolia 024000 (CN); WEI, Aiying, Chifeng, Inner Mongolia 024000 (CN); MENG, Gang, Chifeng, Inner Mongolia 024000 (CN); ZHAO, Chunguang, Chifeng, Inner Mongolia 024000 (CN); SU, Houbo, Chifeng, Inner Mongolia 024000 (CN); YANG, Lipeng, Chifeng, Inner Mongolia 024000 (CN); MA, Fengyong, Chifeng, Inner Mongolia 024000 (CN); ZHOU, Xiaoqun, Chifeng, Inner Mongolia 024000 (CN); GUO, Xiaowei, Chifeng, Inner Mongolia 024000 (CN); TIAN, Bin, Chifeng, Inner Mongolia 024000 (CN)
(74) Representative: ABG Intellectual Property Law, S.L.
(86) International application number: PCT/CN2021/142015
(87) International publication number: WO 2022/143646

(57) **Abstract**

Recombinant strain with modified gene BBD29_09525 for producing L-glutamic acid, and method for constructing the same and use thereof are provided. The recombinant strain is a bacterium that generates L-glutamic acid, and has an improved expression of a polynucleotide encoding an amino acid sequence of SEQ ID NO: 3 or a homologous sequence thereof; the improved expression can be having a point mutation in, and an enhanced expression of the polynucleotide encoding an amino acid sequence of SEQ ID NO: 3 or a homologous sequence thereof. The genetically engineered bacterium is an engineered bacterium that has a mutation of the base at position 337 in a BBD29_09525 gene sequence from cytosine to thymine, causing proline at position 113 in the coded corresponding amino acid sequence to be substituted with serine, and that overexpresses the BBD29_09525 gene or BBD29_09525^{C337T} gene. The genetically engineered bacterium enables increased production and conversion rate of L-glutamic acid.

## Description

### Technical Field

The present invention belongs to the technical field of gene engineering and microorganisms, and in particular relates to recombinant strain for producing L-glutamic acid, and method for constructing the same and use thereof.

### Background Art

L-Glutamic acid is an important amino acid, which is used in food, clinical medicine and other aspects.

Traditionally, L-glutamic acid is mainly produced by fermentation with L-glutamic acid-producing bacteria belonging to the genus *Brevibacterium, Corynebacterium* or *Microtatobiotes,* or the mutants thereof.

L-Glutamic acid is generated by biosynthesis with α-ketoglutaric acid, an intermediate product of citric acid cycle in microbial cells. There are two biosynthetic pathways to form L-glutamic acid from α-ketoglutaric acid through the assimilation of ammonium ions. One pathway is to synthesize L-glutamic acid by catalysis with glutamate dehydrogenase (GDH) in the presence of ammonium ions at a high concentration. The other pathway (GS/GOGAT pathway) is to synthesize L-glutamic acid by glutamine synthetase and glutamine-oxoglutaric acid amino transferase. Glutamine synthetase (GS) catalyzes the conversion of L-glutamic acid and ammonium ions to glutamine; glutamine-oxoglutaric acid amino transferase, also known as "glutamate synthetase" (GOGAT), catalyzes the synthesis of L-glutamic acid, in which two molecules of L-glutamic acid are synthesized from one molecule of glutamine which has been synthesized by GS and one molecule of α-ketoglutaric acid.

The improvement in L-amino acid production by fermentation may relate to fermentation techniques such as stirring and supplying oxygen; or to the composition of the nutrient medium, such as the sugar concentration during fermentation; or to processing the fermentation broth into a product in a suitable form, for example, by drying and pelletizing the fermentation broth or ion exchange chromatography; or may relate to the intrinsic properties of the relevant microorganisms themselves in performance.

Methods for improving the properties of these microorganisms in performance include mutagenesis, mutant selection and screening. The strain obtained in this way is resistant to antimetabolites or auxotrophic for metabolites with regulatory importance and produces L-amino acids, and the like.

Although there are a significant amount of methods capable of enhancing the production capacity of L-glutamic acid, it remains necessary to develop methods to produce L-glutamic acid in order to meet the increasing demand.

### Summary

The objective of the present invention is to develop a new technique for enhancing L-glutamic acid production capacity of a bacterium, thereby providing a method for producing L-glutamic acid effectively.

In order to achieve the above objective, the inventors of the present invention have found in research that the gene BBD29_09525 or a homologous gene thereof in a bacterium can be modified, or improved with respect to the expression thereof to enable an enhanced capacity of the bacterium in L-glutamic acid production. The present invention is completed in view of these discoveries.

The present invention provides a bacterium for generating L-glutamic acid, wherein the expression of a polynucleotide encoding an amino acid sequence of SEQ ID NO: 3 or a homologous sequence thereof is improved. The present invention further provides a method for producing L-glutamic acid with the microorganism.

A first aspect of the present invention provides a bacterium for generating L-glutamic acid, having an improved expression of a polynucleotide encoding an amino acid sequence of SEQ ID NO: 3 or a homologous sequence thereof. According to the present invention, the improved expression is an enhanced expression of the polynucleotide, or having a point mutation in the polynucleotide encoding an amino acid sequence of SEQ ID NO: 3 or a homologous sequence thereof, or having a point mutation in, and an enhanced expression of the polynucleotide encoding an amino acid sequence of SEQ ID NO: 3 or a homologous sequence thereof.

The amino acid sequence of SEQ ID NO: 3 or the homologous sequence thereof is a protein coded by the gene BBD29_09525 or a homologous gene thereof

The bacterium has an enhanced L-glutamic acid production capacity compared with an unmodified strain.

In the present invention, the term "bacterium having L-glutamic acid production capacity" refers to a bacterium having such a capacity of producing and accumulating target L-glutamic acid in a culture medium and/or in a cell of the bacterium that L-glutamic acid can be collected when the bacterium is cultured in the culture medium. A bacterium having L-glutamic acid production capacity can be a bacterium capable of accumulating target L-glutamic acid in a culture medium and/or in a cell of the bacterium in an amount greater than that with an unmodified strain.

The term "unmodified strain" refers to a control strain which has not been modified in a manner such that a specific feature is incorporated. That is, examples of the unmodified strain comprise a wild-type strain and a parent strain.

A bacterium having L-glutamic acid production capacity can be a bacterium capable of accumulating target L-glutamic acid in a culture medium in an amount, preferably above 0.5 g/L, and more preferably above 1.0 g/L.

In the present invention, the term "L-glutamic acid" refers to L-glutamic acid in a free form, a salt thereof or a mixture thereof, unless otherwise specified.

The polynucleotide can encode an amino acid sequence having about 90% or more, about 92% or more, about 95% or more, about 97% or more, about 98% or more, or about 99% or more sequence homology with an amino acid sequence of SEQ ID NO: 3. As used herein, the term "homology" refers to percentage identity between two polynucleotides or two polypeptides as modules. Sequence homology can be measured between one module and another module using a method known in the art. For example, such sequence homology can be measured by virtue of BLAST algorithm.

The expression of a polynucleotide can be enhanced: by substituting or mutating an expression regulatory sequence, introducing a mutation to the sequence of the polynucleotide, and by increasing the copy number of the polynucleotide inserted via a chromosome or introduced with a vector, or a combination thereof, etc.

The expression regulatory sequence of a polynucleotide can be modified. The expression regulatory sequence controls the expression of a polynucleotide operably linked thereto, and can comprise, for example, promoters, terminators, enhancers, silencers and the like. A polynucleotide can have a change in an initiation codon. A polynucleotide can be incorporated into a specific site of a chromosome, thereby increasing copy number. Herein, a specific site can comprise, for example, a transposon site or an intergenic site. In addition, a polynucleotide can be incorporated into an expression vector, and the expression vector is introduced into a host cell, thus increasing copy number.

In an embodiment of the present invention, a polynucleotide, or a polynucleotide having a point mutation is incorporated into a specific site of a chromosome of a microorganism, thus increasing copy number.

In an embodiment of the present invention, a polynucleotide with a promoter sequence, or a polynucleotide having a point mutation with a promoter sequence is incorporated into a specific site of a chromosome of a microorganism, thus overexpressing the nucleic sequence.

In an embodiment of the present invention, a polynucleotide, or a polynucleotide having a point mutation is incorporated into an expression vector, and the expression vector is introduced into a host cell, thus increasing copy number.

In an embodiment of the present invention, a polynucleotide with a promoter sequence, or a polynucleotide having a point mutation with a promoter sequence is incorporated into an expression vector, and the expression vector is introduced into a host cell, thus overexpressing the amino acid sequence.

In a specific embodiment of the present invention, the polynucleotide can comprise a nucleotide sequence of SEQ ID NO: 1.

In an embodiment of the present invention, the polynucleotide encoding an amino acid sequence of SEQ ID NO: 3 has a point mutation such that proline at position 113 in the amino acid sequence of SEQ ID NO: 3 is substituted with a different amino acid.

According to the present invention, preferably proline at position 113 is substituted with serine.

According to the present invention, as set forth in SEQ ID NO: 4 is the amino acid sequence following substitution with serine of proline at position 113 in an amino acid sequence set forth in SEQ ID NO: 3.

In an embodiment of the present invention, the polynucleotide sequence having a point mutation is formed from a mutation to the base at position 337 of a polynucleotide sequence set forth in SEQ ID NO: 1.

According to the present invention, the mutation comprises a mutation of the base at position 337 of a polynucleotide sequence set forth in SEQ ID NO: 1 from cytosine (C) to thymine (T).

In an embodiment of the present invention, the polynucleotide sequence having a point mutation comprises a polynucleotide sequence set forth in SEQ ID NO: 2.

As used herein, the term "operably linked" refers to a functional link between a regulatory sequence and a polynucleotide sequence, whereby the regulatory sequence controls transcription and/or translation of the polynucleotide sequence. A regulatory sequence can be a strong promoter which can enhance the expression level of a polynucleotide. A regulatory sequence can be a promoter derived from a microorganism belonging to the genus *Corynebacterium* or can be a promoter derived from other microorganisms. For example, the promoter can be trc promoter, gap promoter, tac promoter, T7 promoter, lac promoter, trp promoter, araBAD promoter or cj7 promoter.

In a specific embodiment of the present invention, the promoter is a promoter of a polynucleotide encoding an amino acid sequence of SEQ ID NO: 3 (the gene BBD29_09525).

As used herein, the term "vector" refers to a polynucleotide construct containing a regulatory sequence of a gene and a gene sequence and configured to express a target gene in a suitable host cell. Or, a vector can also refer to a polynucleotide construct containing a sequence for homologous recombination such that due to the vector introduced into a host cell, the regulatory sequence of an endogenous gene in the genome of the host cell can be changed, or a target gene that can be expressed can be inserted into a specific site of the genome of a host. In this regard, the vector used in the present invention can further comprise a selective marker to determine the introduction of the vector into a host cell or the insertion of the vector into the chromosome of a host cell. A selective marker can comprise a marker providing a selectable phenotype, such as drug resistance, auxotroph, resistance to cytotoxic agents, or expression of a surface protein. In an environment treated with such a selective agent, the transformed cell can be selected since only the cell that expresses the selective marker can survive or display different phenotypic traits. The vector as described herein is well known to those skilled in the art, including but not limited to: plasmid, bacteriophage (such as λ bacteriophage or M13 filamentous bacteriophage etc.), cosmid (i.e., Cosmid) or viral vector.

In some specific embodiments of the present invention, the vector used is pK18mobsacB plasmid, and pXMJ19 plasmid.

As used herein, the term "transformation" refers to introduction of a polynucleotide into a host cell, such that the polynucleotide can be replication-competent as an element foreign to a genome or by being inserted into the genome of a host cell. A method for transforming the vector used in the present invention can comprise a method for introducing a nucleic acid into a cell. In addition, as disclosed in the related techniques, a method with electric pulse can be performed according to a host cell.

Herein, the microorganism can be yeast, bacterium, alga or fungi.

According to the present invention, the bacterium can be a microorganism belonging to the genus *Corynebacterium,* such as *Corynebacterium acetoacidophilum, Corynebacterium acetoglutamicum*, *Corynebacterium callunae*, *Corynebacterium glutamicum, Brevibacterium flavum, Brevibacterium lactofermentum*, *Corynebacterium ammoniagenes, Corynebacterium pekinense, Brevibacterium saccharolyticum, Brevibacterium roseum,* and *Brevibacterium thiogenitalis,* etc.

In an embodiment of the present invention, the microorganism belonging to the genus *Corynebacterium* is *Corynebacterium glutamicum* ATCC 13869.

In an embodiment of the present invention, the microorganism belonging to the genus *Corynebacterium* is *Corynebacterium glutamicum* YPGLU001, which yields high production of glutamic acid, and was deposited with Strain Name: *Corynebacterium glutamicum;* Latin Name: *Corynebacterium glutamicum;* Strain No.: YPGLU001 in China General Microbiological Culture Collection Center, abbreviated as CGMCC, Address: No. 3, Yard 1, Beichen West Road, Chaoyang District, Beijing on November 23, 2020, with Accession No.: CGMCC No. 21220.

According to the present invention, the bacterium can also have additional improvements relating to the increase in the production of L-glutamic acid, such as enhancement or reduction in the activities of, or in the expressions of the genes of glutamate dehydrogenase, glutamine synthetase, glutamine-oxoglutaric acid amino transferase, etc., or substitution of the genes with foreign genes.

A second aspect of the present invention provides a polynucleotide sequence, an amino acid sequence coded by the polynucleotide sequence, a recombinant vector comprising the polynucleotide sequence, and a recombinant strain containing the polynucleotide sequence.

According to the present invention, the polynucleotide sequence comprises a polynucleotide encoding a polypeptide containing an amino acid sequence set forth in SEQ ID NO: 3with proline at position 113 in the sequence substituted with a different amino acid.

According to the present invention, preferably proline at position 113 is substituted with serine.

According to the present invention, as set forth in SEQ ID NO: 4 is the amino acid sequence following substitution with serine of proline at position 113in an amino acid sequence set forth in SEQ ID NO: 3.

According to the present invention, preferably the polynucleotide encoding a polypeptide containing an amino acid sequence set forth in SEQ ID NO: 3contains a polynucleotide sequence as set forth in SEQ ID NO: 1.

In an embodiment of the present invention, the polynucleotide sequence is formed from a mutation to the base at position 337 in a polynucleotide sequence set forth in SEQ ID NO: 1.

According to the present invention, the mutation refers to a change in the base/nucleotide of the site, and the method for mutation can be at least one selected from mutagenesis, PCR site-directed mutation, and/or homologous recombination, etc. In the present invention, PCR site-directed mutation and/or homologous recombination are preferably used.

According to the present invention, the mutation comprises a mutation of the base at position 337 in a polynucleotide sequence set forth in SEQ ID NO: 1 from cytosine (C) to thymine (T).

In an embodiment of the present invention, the polynucleotide sequence comprises a polynucleotide sequence set forth in SEQ ID NO: 2.

According to the present invention, the amino acid sequence comprises an amino acid sequence set forth in SEQ ID NO: 4.

According to the present invention, the recombinant vector is constructed by introducing the polynucleotide sequence to a plasmid.

In an embodiment of the present invention, the plasmid is pK18mobsacB plasmid.

In another embodiment of the present invention, the plasmid is pXMJ19 plasmid.

Specifically, the polynucleotide sequence and the plasmid can be constructed as a recombinant vector by virtue of NEBuider recombination system.

According to the present invention, the recombinant strain contains the polynucleotide sequence.

As an implementation of the present invention, a starting strain of the recombinant strain is *Corynebacterium glutamicum* CGMCC No. 21220.

As an implementation of the present invention, a starting strain of the recombinant strain is ATCC 13869.

A third aspect of the present invention further provides a method for constructing a recombinant strain generating L-glutamic acid.

According to the present invention, the method for constructing comprises the step of:
engineering a polynucleotide sequence of a wild-type BBD29_09525 gene as set forth in SEQ ID NO: 1 in a host strain to cause a mutation to the base at position 337 to provide a recombinant strain containing a mutated BBD29_09525 coding gene.

According to the method for constructing of the present invention, the engineering comprises at least one of mutagenesis, PCR site-directed mutation, and/or homologous recombination, etc.

According to the method for constructing of the present invention, the mutation refers to a mutation of the base at position 337in SEQ ID NO: 1 from cytosine (C) to thymine (T). Specifically, the polynucleotide sequence containing a mutated BBD29_09525 coding gene is set forth in SEQ ID NO: 2.

Further, the method for constructing comprises the steps of:
(1) engineering a nucleotide sequence of a wild-type BBD29_09525 gene as set forth in SEQ ID NO: 1 to cause a mutation to the base at position 337 to provide a mutated BBD29_09525 gene polynucleotide sequence;
(2) linking the mutated polynucleotide sequence to a plasmid to construct a recombinant vector;
(3) introducing the recombinant vector into a host strain to provide a recombinant strain containing a mutated BBD29_09525 coding gene.

According to the method for constructing of the present invention, the step (1) comprises constructing a BBD29_09525 gene with a point mutation: according to a genome sequence of an unmodified strain, synthesizing two pairs of primers P1, P2 and P3, P4 for amplifying a fragment of the BBD29_09525 gene, and introducing a point mutation to a wild-type BBD29_09525 gene, SEQ ID NO: 1, by virtue of PCR site-directed mutation to provide a BBD29_09525 gene nucleotide sequence with a point mutation, SEQ ID NO: 2, designated as BBD29_09525^{C337T}.

In an embodiment of the present invention, the genome of the unmodified strain can be derived from the strain ATCC13869, the genome sequence of which can be available from the NCBI website.

In an implementation of the present invention, in the step (1), the primers are:

| | |
|---|---|
| P1: | 5' CAGTGCCAAGCTTGCATGCCTGCAGGTCGACTCTAG AAGGAATGTT GTCTGGGCGG 3' (SEQ ID NO: 5) |
| P2: | 5' GGCGTGCTGG CTGATTCTAA GGAATTCATC 3' (SEQ ID NO: 6) |
| P3: | 5' GATGAATTCC TTAGAATCAG CCAGCACGCC 3' (SEQ ID NO: 7) |
| P4: | 5' CAGCTATGACCATGATTACGAATTCGAGCTCGGTACCCGATAGGTCGA TTGTTGGTGT 3' (SEQ ID NO: 8) |

In an implementation of the present invention, the PCR amplification is performed in the following manner: pre-denaturation for 5 min at 94°C, denaturation for 30 s at 94°C, annealing for 30 s at 52°C, and extension for 45 s at 72°C for 30 circles, and overextension for 10 min at 72°C.

In an implementation of the present invention, the overlap PCR amplification is performed in the following manner: pre-denaturation for 5 min at 94°C, denaturation for 30 s at 94°C, annealing for 30 s at 52°C, and extension for 90 s at 72°C for 30 circles, and overextension for 10 min at 72°C.

According to the method for constructing of the present invention, the step (2) comprises constructing a recombinant plasmid, comprising assembling an isolated and purified BBD29_09525^{C337T} with pK18mobsacB plasmid by virtue of NEBuider recombination system to provide a recombinant plasmid.

According to the method for constructing of the present invention, the step (3) comprises constructing a recombinant strain by transforming a recombinant plasmid to a host strain to provide a recombinant strain.

In an implementation of the present invention, the transforming in the step (3) is by an electrotransformation method.

In an embodiment of the present invention, the host strain is ATCC 13869.

In an embodiment of the present invention, the host strain is *Corynebacterium glutamicum* CGMCC No. 21220.

In an embodiment of the present invention, the recombination is achieved by homologous recombination.

A fourth aspect of the present invention further provides a method for constructing a recombinant strain generating L-glutamic acid.

According to the present invention, the method for constructing comprises the steps of:
amplifying the upstream and downstream homology arm fragments of BBD29_09525 and the sequence of a BBD29_09525 gene coding region and a promoter region thereof, and introducing BBD29_09525 or BBD29_09525^{C337T} gene into the genome of a host strain in a manner of homologous recombination so as to enable the strain to overexpress the BBD29_09525 or BBD29_09525^{C337T} gene.

In an embodiment of the present invention, the primers for amplifying the upstream homology arm fragments are:

| | |
|---|---|
| P7: | 5’ CAGTGCCAAGCTTGCATGCCTGCAGGTCGACTCTAG GACCCGCTTG CCATACGAAG |
| | 3' (SEQ ID NO: 11) |
| P8: | 5' CTGAAGCTTG AGGAAGCCTA A ATCTACTCAT CTGAAGAATC 3'(SEQ ID NO: 12) |

In an embodiment of the present invention, the primers for amplifying downstream homologous arm fragments are:
P11: 5' TCGACCTATC GTTATACAT TTCGTGGGCA CTCTGGTTTG 3' (SEQ ID NO: 15)

P12: 5' CAGCTATGACCATGATTACGAATTCGAGCTCGGTACCCCATAAGAAAC
AACCACTTCC 3' (SEQ ID NO: 16)

In an embodiment of the present invention, the primers for amplifying the sequence of the gene coding region and the promoter region thereof are:
P9: 5' GATTCTTCAG ATGAGTAGAT TTAGGCTTCC TCAAGCTTCAG 3' (SEQ ID NO: 13)
P10: 5' CAAACCAGAG TGCCCACGAA ATGTATAACG ATAGGTCGA 3' (SEQ ID NO: 14)

In an embodiment of the present invention, with the above-mentioned P7/P12 as primers again, amplification is performed using as templates a mixture of three fragments, the amplified upstream homology arm fragment, downstream homology arm fragment, and fragment of the sequence of the gene coding region and the promoter region thereof, to provide an integrated homology arm fragment.

In an embodiment of the present invention, the PCR system used comprises: 10 × Ex Taq Buffer 5 µL, dNTP Mixture (2.5 mM each) 4 µL, Mg²⁺ (25 mM) 4 µL, primers (10 pM) 2 µL each, and Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 120 s at 72°C (30 circles), and overextension for 10 min at 72°C.

In an embodiment of the present invention, NEBuider recombination system is used to assemble shuttle plasmid PK18mobsacB with the integrated homology arm fragment to provide an integrative plasmid.

In an embodiment of the present invention, a host strain is transfected with the integrative plasmid to introduce BBD29_09525 or BBD29_09525^{C337T} gene into the genome of the host strain in a manner of homologous recombination.

In an embodiment of the present invention, the host strain is *Corynebacterium glutamicum* CGMCC No. 21220.

In an embodiment of the present invention, the host strain is ATCC 13869.

In an embodiment of the present invention, the host strain is a strain bearing a polynucleotide sequence set forth in SEQ ID NO: 2.

A fifth aspect of the present invention further provides a method for constructing a recombinant strain generating L-glutamic acid.

According to the present invention, the method for constructing comprises the steps of:
amplifying the sequence of BBD29_09525 gene coding region and a promoter region, or the sequence of BBD29_09525^{C337T} gene coding region and a promoter region, constructing an overexpression plasmid vector, and transforming the vector into a host strain to enable the strain to overexpress BBD29_09525 or BBD29_09525^{C337T} gene.

In an embodiment of the present invention, the primers for amplifying the sequence of the gene coding region and the promoter region thereof are:
P17: 5' GCTTGCATGCCTGCAGGTCGACTCTAGAGGATCCCCTTAGGCTTCC TCAAGCTTCAG 3' (SEQ ID NO: 21)
P18: 5' ATCAGGCTGAAAATCTTCTCTCATCCGCCAAAACATGTATAACGATAGGTCGA3'(SEQ ID NO: 22).

In an embodiment of the present invention, the PCR system comprises: 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 120 s at 72°C (30 circles), and overextension for 10 min at 72°C.

In an embodiment of the present invention, NEBuider recombination system is used to assemble shuttle plasmid pXMJ19 with BBD29_09525 or BBD29_09525^{C337T} fragment having its own promoters to provide an overexpression plasmid.

In an embodiment of the present invention, the host strain is *Corynebacterium glutamicum* CGMCC No. 21220.

In an embodiment of the present invention, the host strain is ATCC 13869.

In an embodiment of the present invention, the host strain is a strain bearing a polynucleotide sequence set forth in SEQ ID NO: 2.

The recombinant strain provided in the present invention can be individually applied to fermentation for producing L-glutamic acid, and can also be in hybrid fermentation with other L-glutamic acid-producing bacteria for producing L-glutamic acid.

Another aspect of the present invention provides a method for producing L-glutamic acid, the method comprising culturing the bacterium; and obtaining L-glutamic acid from a culture.

A bacterium can be cultured in a suitable culture medium under culturing conditions as known in the art. The culture medium can comprise: carbon source, nitrogen source, trace elements, and a combination thereof. In culturing, pH of the culture can be adjusted. In addition, bubbles can be prevented from generating in culturing, for example, by using a defoamer to prevent bubbles generation. Further, gases can be injected into the culture in culturing. The gases can comprise any ones capable of maintaining an aerobic condition for the culture. In culturing, the temperature of the culture can be from 20°C to 45°C. The generated L-glutamic acid can be recovered from the culture, i.e., the culture is treated with sulfuric acid or hydrochloric acid, etc. followed by a combination of methods such as anion-exchange chromatography, condensation, crystallization, and isoelectric precipitation.

In the present invention:
SEQ ID NO: 1: BBD29_09525 wild type ORF sequence 
ATGTCAGATT CCCCGAAGAA CGCACCGAGG ATTACCGATG AGGCAGATGT AGTTCTCATT
GGTGCCGGTA TCATGAGCTC CACGCTGGGT GCAATGCTGC GTCAGCTGGA GCCAAGCTGG
ACTCAGATCG TCTTCGAGCG TTTGGATGGA CCGGCACAAG AGTCGTCCTC CCCGTGGAAC
AATGCAGGAA CCGGCCACTC TGCTCTATGC GAGCTGAACT ACACCCCAGA GGTTAAGGGC
AAGGTTGAAA TTGCCAAGGC TGTAGGAATC AACGAGAAGT TCCAGGTTTC CCGTCAGTTC
TGGTCTCACC TCGTTGAAGA GGGCGTGCTG GCTGATCCTA AGGAATTCAT CAACCCTGTT
CCTCACGTAT CTTTCGGCCA GGGCGCAGAC CAGGTTGCAT ACATCAAGGC TCGCTACGAA
GCTTTGAAGG ATCACCCACT CTTCCAGGGC ATGACCTACG CTGACGATGA AGCTACCTTC
ACCGAGAAGC TGCCTTTGAT GGCAAAGGGC CGTGACTTCT CTGATCCAGT AGCAATCTCT
TGGATCGATG AAGGCACCGA CATCAACTAC GGTGCTCAGA CCAAGCAGTA CCTGGATGCA
TCTGAAGTTG AAGGCACTGA AATCCGCTAT GGCCACGAAG TCAAGAGCAT CAAGGCTGAT
 
GGCGCAAAGT GGATCGTGAC CGTCAAGAAC GTACACACTG GCGACACCAA GACCATCAAG
GCAAACTTCG TGTTCGTCGG CGCAGGCGGG TACGCACTGG ATCTGCTTCG CAGCGCAGGC
ATCCCACAGG TCAAGGGCTT CGCTGGATTC CCAGTATCCG GCCTGTGGCT TCGTTGCACC
AACGAGGAAC TGATCGAGCA GCACGCAGCC AAGGTATATG GCAAGGCATC TGTTGGCGCT
CCTCCAATGT CTGTTCCTCA CCTTGACACC CGCGTTATCG AGGGTGAAAA GGGTCTGCTC
TTTGGACCTT ACGGTGGCTG GACCCCTAAG TTCTTGAAGG AAGGCTCCTA CCTGGACCTG
TTCAAGTCCA TCCGCCCAGA CAACATTCCT TCCTACCTTG GCGTTGCTGC TCAGGAATTT
GATCTGACCA AGTACCTTGT CACTGAAGTT CTCAAGGACC AGGACAAGCG TATGGATGCT
CTTCGCGAGT ACATGCCAGA GGCACAAAAC GGCGATTGGG AGACCATCGT TGCCGGACAG
CGTGTTCAGG TTATTAAGCC TGCAGGATTC CCTAAGTTCG GTTCCCTGGA ATTCGGCACC
ACCTTGATCA ACAACTCCGA AGGCACCATC GCCGGATTGC TCGGTGCTTC CCCTGGAGCA
TCCATCGCAC CTTCCGCAAT GATCGAGCTG CTTGAGCGTT GCTTCGGTGA CCGCATGATC
GAGTGGGGCG ACAAGCTGAA GGACATGATC CCTTCCTACG GCAAGAAGCT TGCTTCCGAG
CCAGCACTGT TTGAGCAGCA GTGGGCACGC ACCCAGAAGA CCCTGAAGCT TGAGGAAGCC
TAA
SEQ ID NO: 2: BBD29_09525^{C337T} ORF sequence 
ATGTCAGATT CCCCGAAGAA CGCACCGAGG ATTACCGATG AGGCAGATGT AGTTCTCATT
GGTGCCGGTA TCATGAGCTC CACGCTGGGT GCAATGCTGC GTCAGCTGGA GCCAAGCTGG
ACTCAGATCG TCTTCGAGCG TTTGGATGGA CCGGCACAAG AGTCGTCCTC CCCGTGGAAC
AATGCAGGAA CCGGCCACTC TGCTCTATGC GAGCTGAACT ACACCCCAGA GGTTAAGGGC
AAGGTTGAAA TTGCCAAGGC TGTAGGAATC AACGAGAAGT TCCAGGTTTC CCGTCAGTTC
TGGTCTCACC TCGTTGAAGA GGGCGTGCTG GCTGATTCTA AGGAATTCAT CAACCCTGTT
CCTCACGTAT CTTTCGGCCA GGGCGCAGAC CAGGTTGCAT ACATCAAGGC TCGCTACGAA
GCTTTGAAGG ATCACCCACT CTTCCAGGGC ATGACCTACG CTGACGATGA AGCTACCTTC
ACCGAGAAGC TGCCTTTGAT GGCAAAGGGC CGTGACTTCT CTGATCCAGT AGCAATCTCT
TGGATCGATG AAGGCACCGA CATCAACTAC GGTGCTCAGA CCAAGCAGTA CCTGGATGCA
TCTGAAGTTG AAGGCACTGA AATCCGCTAT GGCCACGAAG TCAAGAGCAT CAAGGCTGAT
GGCGCAAAGT GGATCGTGAC CGTCAAGAAC GTACACACTG GCGACACCAA GACCATCAAG
GCAAACTTCG TGTTCGTCGG CGCAGGCGGG TACGCACTGG ATCTGCTTCG CAGCGCAGGC
ATCCCACAGG TCAAGGGCTT CGCTGGATTC CCAGTATCCG GCCTGTGGCT TCGTTGCACC
AACGAGGAAC TGATCGAGCA GCACGCAGCC AAGGTATATG GCAAGGCATC TGTTGGCGCT
CCTCCAATGT CTGTTCCTCA CCTTGACACC CGCGTTATCG AGGGTGAAAA GGGTCTGCTC
TTTGGACCTT ACGGTGGCTG GACCCCTAAG TTCTTGAAGG AAGGCTCCTA CCTGGACCTG
TTCAAGTCCA TCCGCCCAGA CAACATTCCT TCCTACCTTG GCGTTGCTGC TCAGGAATTT
GATCTGACCA AGTACCTTGT CACTGAAGTT CTCAAGGACC AGGACAAGCG TATGGATGCT
CTTCGCGAGT ACATGCCAGA GGCACAAAAC GGCGATTGGG AGACCATCGT TGCCGGACAG
CGTGTTCAGG TTATTAAGCC TGCAGGATTC CCTAAGTTCG GTTCCCTGGA ATTCGGCACC
ACCTTGATCA ACAACTCCGA AGGCACCATC GCCGGATTGC TCGGTGCTTC CCCTGGAGCA
TCCATCGCAC CTTCCGCAAT GATCGAGCTG CTTGAGCGTT GCTTCGGTGA CCGCATGATC
GAGTGGGGCG ACAAGCTGAA GGACATGATC CCTTCCTACG GCAAGAAGCT TGCTTCCGAG
CCAGCACTGT TTGAGCAGCA GTGGGCACGC ACCCAGAAGA CCCTGAAGCT TGAGGAAGCC
TAA
SEQ ID NO: 3: the amino acid sequence of a protein coded by BBD29_09525 wild type 
MSDSPKNAPR ITDEADVVLI GAGIMSSTLG AMLRQLEPSW TQIVFERLDG PAQESSSPWN
NAGTGHSALC ELNYTPEVKG KVEIAKAVGI NEKFQVSRQF WSHLVEEGVL ADPKEFINPV
 
PHVSFGQGAD QVAYIKARYE ALKDHPLFQG MTYADDEATF TEKLPLMAKG RDFSDPVAIS
WIDEGTDINY GAQTKQYLDA SEVEGTEIRY GHEVKSIKAD GAKWIVTVKN VHTGDTKTIK
ANFVFVGAGG YALDLLRSAG IPQVKGFAGF PVSGLWLRCT NEELIEQHAA KVYGKASVGA
PPMSVPHLDT RVIEGEKGLL FGPYGGWTPK FLKEGSYLDL FKSIRPDNIP SYLGVAAQEF
DLTKYLVTEV LKDQDKRMDA LREYMPEAQN GDWETIVAGQ RVQVIKPAGF PKFGSLEFGT
TLINNSEGTI AGLLGASPGA SIAPSAMIEL LERCFGDRMI EWGDKLKDMI PSYGKKLASE
PALFEQQWAR TQKTLKLEEA
SEQ ID NO: 4: the amino acid sequence of a protein coded by BBD29_09525P113S

MSDSPKNAPR ITDEADVVLI GAGIMSSTLG AMLRQLEPSW TQIVFERLDG PAQESSSPWN
NAGTGHSALC ELNYTPEVKG KVEIAKAVGI NEKFQVSRQF WSHLVEEGVL ADSKEFINPV
PHVSFGQGAD QVAYIKARYE ALKDHPLFQG MTYADDEATF TEKLPLMAKG RDFSDPVAIS
WIDEGTDINY GAQTKQYLDA SEVEGTEIRY GHEVKSIKAD GAKWIVTVKN VHTGDTKTIK
ANFVFVGAGG YALDLLRSAG IPQVKGFAGF PVSGLWLRCT NEELIEQHAA KVYGKASVGA
PPMSVPHLDT RVIEGEKGLL FGPYGGWTPK FLKEGSYLDL FKSIRPDNIP SYLGVAAQEF
DLTKYLVTEV LKDQDKRMDA LREYMPEAQN GDWETIVAGQ RVQVIKPAGF PKFGSLEFGT
TLINNSEGTI AGLLGASPGA SIAPSAMIEL LERCFGDRMI EWGDKLKDMI PSYGKKLASE
PALFEQQWAR TQKTLKLEEA

BBD29_09525^{P113S} is BBD29_09525 P113S.

The present invention also provides a protein, designated as protein BBD29_09525^{P113S}, wherein the protein can be any one of:
A1) a protein whose amino acid sequence is SEQ ID NO: 4;
A2) a protein having 80% or more identity to and the same function as the protein indicated in A1), as obtained by subjecting the amino acid sequence set forth in SEQ ID NO: 4 to substitution and/or deletion and/or addition of amino acid residues;
A3) a fusion protein having the same function, as obtained by linking a tag to the N-terminus and/or C-terminus of A1) or A2).

The present invention also provides a nucleic acid molecule, designated as BBD29_09525^{C337T}, wherein the nucleic acid molecule BBD29_09525^{C337T} can be any one of:
B1) a nucleic acid molecule encoding the protein BBD29_09525^{P113S};
B2) a DNA molecule whose coding sequence is set forth in SEQ ID NO: 2;
B3) a DNA molecule whose nucleotide sequence is set forth in SEQ ID NO: 2.

The DNA molecule set forth in SEQ ID NO: 2 is the BBD29_09525^{C337T} gene of the present invention.

The DNA molecule set forth in SEQ ID NO: 2 (the BBD29_09525^{C337T} gene) encodes the protein BBD29_09525^{P113S} set forth in SEQ ID NO: 4.

Serine (S) at position 113 in an amino acid sequence of the protein BBD29_09525^{P113S} (SEQ ID NO: 4) is derived from a mutation of proline (P).

The present invention also provides a biomaterial, wherein the biomaterial can be any one of:
C1) an expression cassette comprising the nucleic acid molecule BBD29_09525^{C337T} ;
C2) a recombinant vector comprising the nucleic acid molecule BBD29_09525^{C337T} or a recombinant vector comprising the expression cassette of C1);
C3) a recombinant microorganism comprising the nucleic acid molecule BBD29_09525^{C337T}, or a recombinant microorganism comprising the expression cassette of C1), or a recombinant microorganism comprising the recombinant vector of C2).

The present invention also provides any one of the following uses of any one of D1)-D8):
F1) use of any one of D1)-D8) in the regulation of the production of L-glutamic acid of a microorganism;
F2) use of any one of D1)-D8) in the construction of a genetically engineered bacterium for producing L-glutamic acid;
F3) use of any one of D1)-D8) in the preparation of L-glutamic acid;
wherein the D1)-D8) is:
   D1) the protein BBD29_09525^{P113S};
   D2) the nucleic acid molecule BBD29_09525^{C337};
   D3) the biomaterial;
   D4) a DNA molecule whose nucleotide sequence is SEQ ID NO: 1;
   D5) a DNA molecule having 90% or more identity to and the same function as the DNA molecule set forth in SEQ ID NO: 1, as obtained by subjecting the nucleotide sequence set forth in SEQ ID NO: 1 to modification, and/or substitution and/or deletion and/or addition of one or several nucleotides;
   D6) an expression cassette comprising the DNA molecule in D4) or D5);
   D7) a recombinant vector comprising the DNA molecule in D4) or D5), or a recombinant vector comprising the expression cassette of D6);
   D8) a recombinant microorganism comprising the DNA molecule in D4) or D5), or a recombinant microorganism comprising the expression cassette of D6), or a recombinant microorganism comprising the recombinant vector of D7).

The DNA molecule set forth in SEQ ID NO: 1 is the BBD29_09525 gene of the present invention.

The DNA molecule set forth in SEQ ID NO: 1 (the BBD29_09525 gene) encodes a protein set forth in SEQ ID NO: 3.

Herein, identity refers to the identity between amino acid sequences or nucleotide sequences. International homology retrieval sites on the Internet can be employed to determine the identity between amino acid sequences, such as the BLAST page on the NCBI homepage website. For example, in Advanced BLAST2.1, blastp can be used as a program, with the Expect value set at 10, each Filter set to OFF, BLOSUM62 used as Matrix, and Gap existence cost, Per residue gap cost and Lambda ratio set at 11, 1 and 0.85 (default values), respectively, and a search is done for the identity between a pair of amino acid sequences for calculation, and then a value of identity (%) can be available.

Herein, the 80% or more identity can be an identity of at least 80%, 81%, 82%, 83%, 84%, 85%, 86%, 87%, 88%, 89%, 90%, 91%, 92%, 93%, 94%, 95%, 96%, 97%, 98% or 99%.

Herein, the 90% or more identity can be an identity of at least 90%, 91%, 92%, 93%, 94%, 95%, 96%, 97%, 98% or 99%.

The regulation of the production of L-glutamic acid of a microorganism as described herein can be increasing or reducing the amount of the accumulation of L-glutamic acid in the microorganism (i.e., promoting or inhibiting the biosynthesis of L-glutamic acid).

The present invention also provides a method for increasing the production of L-glutamic acid in a microorganism, the method comprising any one of:
E1) increasing the expression amount, or content of the nucleic acid molecule BBD29_09525^{C337T} in a target microorganism to provide a microorganism having a greater production of L-glutamic acid than the target microorganism;
E2) increasing the expression amount, or content of the DNA molecule of D4) or D5) in a target microorganism to provide a microorganism having a greater production of L-glutamic acid than the target microorganism;
E3) performing a mutation on the DNA molecule whose nucleotide sequence is SEQ ID NO: 1 in the target microorganism to provide a microorganism having a greater production of L-glutamic acid than the target microorganism.

In the method above, the mutation can be a point mutation, i.e., a mutation of a single nucleotide.

In the method above, the point mutation can be a mutation of proline residue at position 113 in an amino acid sequence coded by the DNA molecule set forth in SEQ ID NO: 1 to another amino acid residue.

In the method above, the point mutation can be a mutation of proline at position 113 in an amino acid sequence coded by the DNA molecule set forth in SEQ ID NO: 1 to serine, providing a mutated protein BBD29_09525^{P113S} whose amino acid sequence is SEQ ID NO: 4.

The mutation refers to a change in one or several bases in a gene through site-directed mutation, resulting in a change in the constitution of the amino acids of a corresponding protein, thereby generating a new protein or causing a new function to the original protein, that is, site-directed mutation to genes. Techniques for site-directed mutation to genes such as oligonucleotide primer-mediated site-directed mutation, PCR-mediated site-directed mutation, or cassette mutation are well known to those skilled in the art.

The point mutation as described herein can be replacement of a single base, insertion of a single base, or deletion of a single base, and specifically, replacement of a single base. The replacement of a single base can be allelic replacement.

The point mutation can be performing a nucleic acid modification to cytosine (C) at position 337 of the BBD29_09525 gene (SEQ ID NO: 1).

Specifically, the point mutation can be performing a mutation to cytosine (C) at position 337 of the BBD29_09525 gene (SEQ ID NO: 1) to thymine (T) to provide a DNA molecule set forth in SEQ ID NO: 2.

Herein, the recombinant vector can be, specifically, recombinant vector pK18-BBD29_09525^{C337T}, PK18mobsacB-BBD29_09525, PK18mobsacB-BBD29_09525^{C337T}, pXMJ19-BBD29_09525 or pXMJ19-BBD29_09525^{C337T}.

The recombinant vector pK18-BBD29_09525^{C337T} is a recombinant vector obtained by substituting a fragment (a small fragment) between *Xbal* I and/*BamH* I recognition sites in pK18mobsacB vector with the DNA fragment set forth as positions 37-1466 of SEQ ID NO: 29 in the Sequence Listing, while keeping the other sequences of the pK18mobsacB vector unchanged. The recombinant vector pK18-BBD29_09525^{C337T} contains a DNA molecule set forth as positions 1-1051 in the mutated gene BBD29_09525^{C337T} as set forth in SEQ ID NO: 2.

The recombinant vector PK18mobsacB-BBD29_09525 is used to integrate an exogenous gene BBD29_09525 into a chromosome of a host so as to overexpress a wild-type BBD29_09525 gene in a producer bacterium. The recombinant vector pK18mobsacB-BBD29_09525 is a recombinant vector obtained by substituting a fragment (a small fragment) between *Xbal* I and/*BamH* I recognition sites in pK18mobsacB vector with the DNA fragment set forth as positions 37-3407 of SEQ ID NO: 30 in the Sequence Listing, while keeping the other sequences of the pK18mobsacB vector unchanged.

The recombinant vector PK18mobsacB-BBD29_09525^{C337T} is used to integrate an exogenous gene BBD29_09525^{C337T} into a chromosome of a host so as to overexpress a mutant-type gene BBD29_09525^{C337T} in a producer bacterium. The recombinant vector pK18mobsacB-BBD29_09525^{C337T} is a recombinant vector obtained by substituting a fragment (a small fragment) between *Xbal* I and/*BamH* I recognition sites in pK18mobsacB vector with the DNA fragment set forth as positions 37-3407 of SEQ ID NO: 31 in the Sequence Listing, while keeping the other sequences of the pK18mobsacB vector unchanged.

The recombinant vector pXMJ19-BBD29_09525 is used to express via a plasmid an exogenous gene BBD29_09525 outside a chromosome so as to overexpress a wild-type BBD29_09525 gene in a producer bacterium. The recombinant vector pXMJ19-BBD29_09525 is a recombinant vector obtained by substituting a fragment (a small fragment) between *EcoR* I and *Kpn* I recognition sites in pXMJ19 vector with the DNA fragment set forth as positions 37-1927 of SEQ ID NO: 32 in the Sequence Listing, while keeping the other sequences of the pXMJ19 vector unchanged.

The recombinant vector pXMJ19-BBD29_09525^{C337T} is used to express via a plasmid an exogenous gene BBD29_09525^{C337T} outside a chromosome so as to overexpress a mutant-type gene BBD29_09525^{C337T} in a producer bacterium. The recombinant vector pXMJ19-BBD29_09525^{C337T} is a recombinant vector obtained by substituting a fragment (a small fragment) between *EcoR* I and *Kpn* I recognition sites in pXMJ19 vector with the DNA fragment set forth as positions 37-1927 of SEQ ID NO: 33 in the Sequence Listing, while keeping the other sequences of the pXMJ19 vector unchanged.

The recombinant vectors pK18-BBD29_09525^{C337T}, PK18mobsacB-BBD29_09525, PK18mobsacB-BBD29_09525^{C337T}, pXMJ19-BBD29_09525 and pXMJ19-BBD29_09525^{C337T} are all within the scope of protection of the present invention.

Herein, the recombinant microorganism can be, specifically, a recombinant bacterium YPG-007, YPG-008, YPG-009, YPG-010 or YPG-011.

The recombinant bacterium YPG-007 is a recombinant bacterium obtained by transformation of the recombinant vector pK18-BBD29_09525^{C337T} into *Corynebacterium glutamicum* CGMCC No. 21220, and the recombinant bacterium YPG-007 contains the mutated gene BBD29_09525^{C337T} set forth in SEQ ID NO: 2.

The recombinant bacterium YPG-008 contains a double-copy BBD29_09525 gene set forth in SEQ ID NO: 1; a recombinant bacterium containing a double-copy BBD29_09525 gene can significantly and steadily increase the expression amount of BBD29_09525 gene. The recombinant bacterium YPG-008 is an engineered bacterium overexpressing a wild-type BBD29_09525 gene on genome.

The recombinant bacterium YPG-009 contains a mutated BBD29_09525^{C337T} gene set forth in SEQ ID NO: 2; the recombinant bacterium YPG-009 is an engineered bacterium overexpressing a mutant-type BBD29_09525^{C337T} gene on genome.

The recombinant bacterium YPG-010 contains a double-copy BBD29_09525 gene set forth in SEQ ID NO: 1; the recombinant bacterium YPG-010 is an engineered bacterium overexpressing a wild-type BBD29_09525 gene on a plasmid, i.e., overexpression outside a chromosome via a plasmid pXMJ19-BBD29_09525.

The recombinant bacterium YPG-011 contains a mutated BBD29_09525^{C337T} gene set forth in SEQ ID NO: 2; the recombinant bacterium YPG-011 is an engineered bacterium overexpressing a mutant-type BBD29_09525^{C337T} gene on a plasmid, i.e., overexpression outside a chromosome via a plasmid pXMJ19-BBD29_09525^{C337T}.

The recombinant bacteria YPG-007, YPG-008, YPG-009, YPG-010 and YPG-011 are all within the scope of protection of the present invention.

The present invention also provides a method for constructing the recombinant microorganism, the method comprising at least one of:
F1) introducing the nucleic acid molecule BBD29_09525^{C337T} into a target microorganism to provide the recombinant microorganism;
F2) introducing the DNA molecule set forth in SEQ ID NO: 1 into a target microorganism to provide the recombinant microorganism;
F3) editing the DNA molecule set forth in SEQ ID NO: 1 with a gene editing measure (such as single-base gene editing) to contain the DNA molecule set forth in SEQ ID NO: 2 in a target microorganism.

The introducing can be host bacterium transformation with a vector bearing a DNA molecule of the present invention through any of known transformation methods, such as a chemical transformation method or an electrotransformation method. The introduced DNA molecule can be single-copy, or multi-copy. The introducing can be integration of an exogenous gene into a chromosome of a host, or expression outside a chromosome via a plasmid.

The present invention also provides a method for preparing L-glutamic acid, the method comprising producing L-glutamic acid with any one of the recombinant microorganisms as described herein.

In the method above, the method can be preparation of L-glutamic acid in a fermentation method, and the recombinant microorganism can be the genus *Corynebacterium,* and specifically, *Corynebacterium glutamicum* and variants thereof.

Depositary Information: Strain Name: *Corynebacterium glutamicum*; Latin Name: *Corynebacterium glutamicum*; Strain No.: YPGLU001; Depositary Institution: China General Microbiological Culture Collection Center, abbreviated as CGMCC; Address: No. 3, Yard 1, Beichen West Road, Chaoyang District, Beijing; Depositary Date: November 23, 2020; Accession No. at the depositary center: CGMCC No. 21220.

### Best Modes for Carrying out the Invention

The present invention will be further described in detail in connection to specific embodiments, and the examples are given only to illustrate the present invention, but not to limit the scope of the present invention. The examples provided below can be used as a guide for further improvement by those skilled in the art, and are not intended to limit the invention in any way.

Unless otherwise specified specially, the experimental methods in the following examples are all routine methods, which are carried out according to the techniques or conditions described in the literature in the art, or according to the instructions of products. Unless otherwise specified specially, the materials and reagents used in the following examples can be commercially available.

In the following examples, the constitution of the basic medium used to culture the strain is the same, and sucrose, kanamycin or chloramphenicol are added based on the constitution of the basic medium for a corresponding demand. The constitution of the basic medium is shown in Table 1:

**Table 1 Constitution of Basic Culture Medium**

| Ingredients | Formulation |
|---|---|
| Sucrose | 10 g/L |
| Polypeptone | 10 g/L |
| Beef Extract | 10 g/L |
| Powdered Yeast | 5 g/L |
| Urea | 2 g/L |
| Sodium Chloride | 2.5 g/L |
| Powdered Agar | 20 g/L |
| pH | 7.0 |
| Temperature for Culturing | 32 Degrees |

*Corynebacterium glutamicum* YPGLU001 CGMCC No. 21220 in the following examples has been deposited at China General Microbiological Culture Collection Center (abbreviated as CGMCC, Address: No. 3, Yard 1, Beichen West Road, Chaoyang District, Beijing, Institute of Microbiology, Chinese Academy of Sciences) on November 23, 2020, with Accession No. CGMCC No. 21220. *Corynebacterium glutαmicum*YPGLU001 is also known as *Corynebacterium glutamicum* CGMCC No. 21220.

### Example 1. Construction of Transformation Vector pK18-BBD29_09525^{C337T} Comprising BBD29_09525 Gene Coding Region with Point Mutation

According to the genome sequence of *Corynebacterium glutamicum* ATCC13869 published on NCBI, two pairs of primers for amplifying the sequence of BBD29_09525 gene coding region are designed and synthesized, and a point mutation is introduced into a strain, *Corynebacterium glutamicum* CGMCC No. 21220, in allelic replacement (the BBD29_09525 gene coding region on the chromosome of the strain is confirmed consistent with that of ATCC13869 upon sequencing). The amino acid sequence corresponding to a coded protein is SEQ ID NO: 3, with a change of cytosine (C) at position 337 in the nucleotide sequence of BBD29_09525 gene to thymine (T) (SEQ ID NO: 2: BBD29_09525^{C337T}), and thus a change of proline (P) at position 113 in the amino acid sequence corresponding to the coded protein to serine (S) (SEQ ID NO: 4: BBD29_09525 P113S).

The point mutation is a mutation of cytosine (C) at position 337 in a nucleotide sequence of BBD29_09525 gene (SEQ ID NO: 1) to thymine (T), providing a DNA molecule set forth in SEQ ID NO: 2 (a mutated BBD29_09525 gene, designated as BBD29_09525^{C337T}).

Wherein, the DNA molecule set forth in SEQ ID NO: 1 encodes a protein whose amino acid sequence is SEQ ID NO: 3 (the protein is designated as protein BBD29_09525).

The DNA molecule set forth in SEQ ID NO: 2 encodes a mutant protein whose amino acid sequence is SEQ ID NO: 4 (the mutant protein is designated as BBD29_09525^{P113S}). Serine (S) at position 113 in an amino acid sequence of the mutant protein BBD29_09525^{P113S} (SEQ ID NO: 4) is derived from a mutation of proline (P).

Overlap PCR technique is employed for site-directed mutation to genes, with the primers designed as follows (synthesized by Invitrogen Corporation, Shanghai). The bold base is where mutation occurs:

| | |
|---|---|
| P1: | 5' CAGTGCCAAGCTTGCATGCCTGCAGGTCGACTCTAG AAGGAATGTT GTCTGGGCGG 3' (SEQ ID NO: 5) |
| P2: | 5' GGCGTGCTGG CTGATTCTAA GGAATTCATC 3' (SEQ ID NO: 6) |
| P3: | 5' GATGAATTCC TTAGAATCAG CCAGCACGCC 3' (SEQ ID NO: 7) |
| P4: | 5' CAGCTATGACCATGATTACGAATTCGAGCTCGGTACCCGATAGGTCGA TTGTTGGTGT 3' (SEQ ID NO: 8) |

Method for Construction: using *Corynebacterium glutamicum* ATCC13869 as a template, PCR amplification is performed with respective primers P1, P2 and P3, P4 to provide two DNA fragments of a BBD29_09525 gene coding region each having a mutated base and sized in 766 bp and 768 bp, respectively (BBD29_09525 Up and BBD29_09525 Down).

PCR System: 10×Ex Taq Buffer 5 µL, dNTP Mixture (2.5 mM each) 4 µL, Mg²⁺(25mM) 4 µL, primers (10 pM) 2 µL each, Ex Taq (5U/µL) 0.25 µL in a total volume of 50 µL.

The above PCR amplification is performed by pre-denaturation for 5min at 94°C, denaturation for 30s at 94°C, annealing for 30s at 52°C, extension for 45s at 72°C for 30 circles, and overextension for 10min at 72°C, to provide two DNA fragments containing a BBD29_09525 gene coding region each sized in 766 bp and 768 bp (BBD29_09525 Up and BBD29_09525 Down).

Target bands are recovered after the above two DNA fragments (BBD29_09525 Up and BBD29_09525 Down) are isolated via Agarose Gel Electrophoresis and purified. The above two DNA fragments are then used as a template with P1 and P4 as primers for overlap PCR amplification to provide a fragment in 1504bp in length, designated as BBD29_09525 Up-Down (as set forth in SEQ ID NO: 29 for the sequence). In the DNA molecule set forth in SEQ ID NO: 29, positions 37-1087 are a BBD29_09525^{C337T} gene fragment with a mutation site (i.e., positions 1-1051 of SEQ ID NO: 2).

Overlap PCR System: 10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL in a total volume of 50 µL.

The overlap PCR amplification is performed by pre-denaturation for 5min at 94°C, denaturation for 30s at 94°C, annealing for 30s at 52°C, extension for 90s at 72°C for 30 circles, and overextension for 10min at 72°C.

The DNA fragment BBD29_09525 Up-Down (SEQ ID NO: 29) contains a mutation site for introducing nucleic acid modification to position 337 in a BBD29_09525 gene coding region in *Corynebacterium glutamicum* CGMCC No. 21220, specifically, a change of cytosine (C) at position 337 in the BBD29_09525 gene coding region in the *Corynebacterium glutamicum* CGMCC No. 21220 to thymine (T), and finally resulting in change of the amino acid at position 113 of a coded protein from proline (P) to serine (S).

After pK18mobsacB plasmid (purchased from Addgene Coporation) is cleaved with *Xba* I/*BαmH* I*,* BBD29_09525^{C337T} and the linearized pK18mobsacB plasmid are isolated via Agarose Gel Electrophoresis and purified, followed by assembly using NEBuider recombination system to provide vector pK18-BBD29_09525^{C337T}, a plasmid containing a kanamycin-resistant marker. And, the vector pK18-BBD29_09525^{C337T} is sent for sequencing and identification in a company for sequencing, while vector pK18-BBD29_09525^{C337T} containing a correct point mutation (C-T) is preserved for reserve.

Specifically, the DNA fragment (BBD29_09525 Up-Down) is purified after being isolated via Agarose Gel Electrophoresis, followed by ligation to a pK18mobsacB plasmid (purchased from Addgene Corporation, cleaved with *Xbal* I/*BamH* I) purified after being cleaved with enzymes (*Xbal* I/*BamH* I) for 30 min at 50°C using NEBuilder enzyme (purchased from NEB Corporation). A grown single clone after the ligation product is transformed into DH5a (purchased form TAKARA Corporation) is subjected to PCR identification for a positive recombinant vector pK18-BBD29_09525^{C337T}, a recombinant vector containing a kanamycin-resistant (Kan^{r}) marker. A recombinant vector pK18- BBD29_09525^{C337T} with correct cleavage is sent for sequencing and identification in a company for sequencing, while a recombinant vector pK18-BBD29_09525^{C337T} containing a correct point mutation (C-T) is preserved for reserve.

The recombinant vector pK18-BBD29_09525^{C337T} is a recombinant vector obtained by substituting a fragment (a small fragment) between *Xbal* I and/*BamH* I recognition sites in pK18mobsacB vector with the DNA fragment set forth as positions 37-1466 of SEQ ID NO: 29 in the Sequence Listing, while keeping the other sequences of the pK18mobsacB vector unchanged.

The recombinant vector pK18-BBD29_09525^{C337T} contains a DNA molecule set forth as positions 1-1051 in the mutated gene BBD29_09525^{C337T} set forth in SEQ ID NO: 2

### Example 2. Construction of Engineered Strain Comprising BBD29_09525^{C337T} with Point Mutation

Method for Construction: Plasmid pK18-BBD29_09525^{C337T} following allelic replacement in Example 1 is electrotransformed into *Corynebacterium glutamicum* CGMCC No. 21220 for culturing in a culturing medium. See Table 1 for the ingredients in the medium and the conditions for culturing. The cultured mono-colonies are each identified with primer P1 and universal primer M13R for a positive strain with a band in about 1511 bp following amplification. The positive strain is cultured on a culture medium containing 15% sucrose, and the cultured mono-colonies are cultured on culture media with and without kanamycin, respectively. Strains that grow on a culture medium without kanamycin and do not grow on a culture medium with kanamycin are selected for further identification by PCR with the primers as follows (synthesized by Invitrogen Corporation, Shanghai):

| | |
|---|---|
| P5: | 5' CATCAAAGGC AGCTTCTCGG 3' (SEQ ID NO: 9) |
| P6: | 5' ACTACACCCC AGAGGTTAAG 3' (SEQ ID NO: 10) |

The above amplified products from PCR are subjected to denaturation at a high temperature and ice bath before SSCP electrophoresis (with an amplified fragment of vector pK18-BBD29_09525^{C337T} as a positive control, an amplified fragment of ATCC13869 as a negative control, and water as a blank control). See Table 2 for the preparation of PAGE for SSCP electrophoresis and the conditions for electrophoresis. Since fragments are different in their structures, their locations in electrophoresis are different. Thus, a strain having successful allelic replacement is such a strain that the location of its fragment in electrophoresis is inconsistent with that of a negative control fragment and consistent with that of a positive control fragment. Primers P5 and P6 are used again for PCR amplification of a target fragment of the strain with successful allelic replacement, which is then ligated to PMD19-T vector for sequencing. By virtue of sequence alignment, the sequence with a mutated base sequence verified that the allelic replacement of the strain is successful, which is designated as YPG-007.

The recombinant bacterium YPG-007 contains a mutated gene BBD29_09525^{C337T} set forth in SEQ ID NO: 2.

**Table 2 Preparation of PAGE and Conditions for SSCP Electrophoresis**

| Ingredients | Amount (Final Concentration of 8% for Acrylamide Formulation) |
|---|---|
| 40% Acrylamide | 8 mL |
| ddH₂O | 26 mL |
| Glycerol | 4 mL |
| 10×TBE | 2 mL |
| TEMED | 40 µL |
| 10%APS | 600 µL |
| Conditions for Electrophoresis | Electrophoresis Tank Placed into Ice, 1 ×TBE Buffer, Voltage 120V for 10h |

### Example 3. Construction of Engineered Strain with BBD29_09525 or BBD29_09525^{C337T} Gene Overexpressing on Genome

In order to further investigate and verify that overexpressing a wild-type BBD29_09525 gene or a mutant gene thereof, BBD29_09525^{C337T}, in a producer bacterium can increase the production of L-glutamic acid, an exogenous gene is integrated into a chromosome of a host to construct an engineered strain with BBD29_09525 gene or BBD29_09525^{C337T} gene overexpressing on genome.

According to the genome sequence of *Corynebacterium glutamicum* ATCC13869 published on NCBI, three pairs of primers are designed and synthesized for amplification of upstream and downstream homology arm fragments, and a sequence of BBD29_09525 gene coding region and a promoter region for introduction of BBD29_09525 or BBD29_09525^{C337T} gene into the strain *Corynebacterium glutamicum* CGMCC No. 21220 through homologous recombination.

The primers are designed as follows (synthesized by Invitrogen Corporation, Shanghai):

| | |
|---|---|
| P7: | 5' CAGTGCCAAGCTTGCATGCCTGCAGGTCGACTCTAG GACCCGCTTG CCATACGAAG 3' (SEQ ID NO: 11) |
| P8: | 5' CTGAAGCTTG AGGAAGCCTA A ATCTACTCAT CTGAAGAATC 3' (SEQ ID NO: 12) |
| P9: | 5' GATTCTTCAG ATGAGTAGAT TTAGGCTTCC TCAAGCTTCAG 3' (SEQ ID NO: 13) |
| P10: | 5' CAAACCAGAG TGCCCACGAA ATGTATAACG ATAGGTCGA 3' (SEQ ID NO: 14) |
| P11: | 5' TCGACCTATC GTTATACAT TTCGTGGGCA CTCTGGTTTG 3' (SEQ ID NO: 15) |
| P12: | 5' CAGCTATGACCATGATTACGAATTCGAGCTCGGTACCCCATAAGAAAC AACCACTTCC 3' (SEQ ID NO: 16) |

Method for Construction: Using *Corynebacterium glutamicum* ATCC13869 or YPG-007, respectively, as a template, PCR amplification is performed with primers P7/P8, P9/P10, and P11/P22, respectively, to provide an upstream homologous arm fragment in about 807bp, a fragment of BBD29_09525 gene coding region and promoter region in 1931bp or a fragment of BBD29_09525^{C337T} gene coding region and promoter region in about 1931bp, and a downstream homologous arm fragment in about 787bp. Further, using P7/P12 as primers, amplification is performed with a mixture of the three amplified fragments above as a template to provide integrated homologous arm fragments 1 and 2 (integrated homologous arm fragment 1 in 3445bp, as set forth in SEQ ID NO: 30 for its sequence; and integrated homologous arm fragment 2 in 3445bp, as set forth in SEQ ID NO: 31 for its sequence). After the PCR reaction is completed, electrophoresis is performed on the amplified products for recovering a desired DNA fragment in about 3445bp using a Column DNA Gel Recovery Kit (TIANGEN), which is ligated, using NEBuider recombination system, to shuttle plasmid PK18mobsacB having been cleaved with *Xbal* and recovered to provide an integrative plasmid (i.e., a recombinant vector) PK18mobsacB-BBD29_09525 or PK18mobsacB-BBD29_09525^{C337T}. The plasmid contains a kanamycin-resistant marker through which a recombinant having a plasmid integrated into genome can be obtained by screening with kanamycin.

The recombinant vector pK18mobsacB-BBD29_09525 is a recombinant vector obtained by substituting a fragment (a small fragment) between *Xbal* I and/*BαmH* I recognition sites in pK18mobsacB vector with the DNA fragment set forth as positions 37-3407 of SEQ ID NO: 30 in the Sequence Listing, while keeping the other sequences of the pK18mobsacB vector unchanged.

The recombinant vector pK18mobsacB-BBD29_09525^{C337T} is a recombinant vector obtained by substituting a fragment (a small fragment) between *Xbal* I and/*BamH* I recognition sites in pK18mobsacB vector with the DNA fragment set forth as positions 37-3407 of SEQ ID NO: 31 in the Sequence Listing, while keeping the other sequences of the pK18mobsacB vector unchanged.

PCR System: 10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL in a total volume of 50 µL.

The PCR amplification is performed by pre-denaturation for 5min at 94°C, denaturation for 30s at 94°C, annealing for 30s at 52°C, and extension for 120s at 72°C (30 cycles), and overextension for 10min at 72°C.

The two integrative plasmids (PK18mobsacB-BBD29_09525 and PK18mobsacB-BBD29_09525^{C337T}) are each introduced into the strain *Corynebacterium glutamicum* CGMCC No. 21220 by virtue of electrotransformation, and PCR identification is performed on the cultured mono-colonies with primers P13/P14 for a positive strain containing a fragment in about 1821 bp from PCR amplification, while a strain without any fragments amplified therefrom is an original one. Having being screened with 15% sucrose, the positive strain is cultured on culture media with and without kanamycin, respectively, and strains that grow on a culture medium without kanamycin and do not grow on a culture medium with kanamycin are further subjected to PCR for identification with primers P15/P16. The strain with a fragment amplified therefrom in about 1769 bp is a strain having BBD29_09525 or BBD29_09525^{C337T} gene integrated into the genome of *Corynebacterium glutamicum* CGMCC No. 21220, designated as YPG-008 (without a mutation point) and YPG-009 (with a mutation point).

The recombinant bacterium YPG-008 contains a double-copy BBD29_09525 gene as set forth in SEQ ID NO: 1; the recombinant bacterium containing the double-copy BBD29_09525 gene can significantly and steadily increase the expression amount of BBD29_09525 gene. The recombinant bacterium YPG-008 is an engineered bacterium overexpressing a wild-type BBD29_09525 gene on genome.

The recombinant bacterium YPG-009 contains a mutated BBD29_09525^{C337T} gene as set forth in SEQ ID NO: 2; the recombinant bacterium YPG-009 is an engineered bacterium overexpressing a mutant BBD29_09525^{C337T} gene on genome.

The primers for PCR identification are shown as follows:
P13: 5' GTCCAAGGTG ACGGCCGCAC 3' (SEQ ID NO: 17)
P14: 5' TGACTTCTCT GATCCAGTAG 3' (SEQ ID NO: 18)
P15: 5' GTACTGCTTG GTCTGAGCAC 3' (SEQ ID NO: 19)
P16: 5' ATATTCGGCC CAGCAGCAGC 3' (SEQ ID NO: 20)

### Example 4. Construction of Engineered Strain Overexpressing BBD29_09525 or BBD29_09525^{C337T} Gene on Plasmid

According to the genome sequence of *Corynebacterium glutamicum* ATCC13869 published on NCBI, a pair of primers for amplifying a sequence of BBD29_09525 gene coding region and promoter region are designed and synthesized. The primers are designed as follows (synthesized by Invitrogen Corporation, Shanghai):
P17: 5' GCTTGCATGCCTGCAGGTCGACTCTAGAGGATCCCCTTAGGCTTCC TCAAGCTTCAG 3' (SEQ ID NO: 21)
P18: 5' ATCAGGCTGAAAATCTTCTCTCATCCGCCAAAACATGTATAACGATAGGTCGA 3' (SEQ ID NO: 22)

Method for Construction: Using YPG-008 or YPG-007, respectively, as a template, PCR amplification is performed with primers P17/P18 to provide a fragment of BBD29_09525 gene and the promoter thereof in 1961 bp (SEQ ID NO: 32) or a fragment of BBD29_09525^{C337T} gene and the promoter thereof in 1961 bp (SEQ ID NO: 33). Electrophoresis is performed on the amplified products for recovering a desired DNA fragment in 1961 bp using a Column DNA Gel Recovery Kit, which is ligated, using NEBuider recombination system, to shuttle plasmid pXMJ19 having been cleaved with *EcoR* I/*Kpn* I and recovered to provide an overexpression plasmid (i.e., a recombinant vector) pXMJ19-BBD29_09525 or pXMJ19-BBD29_09525^{C337T}. The plasmid contains a chloramphenicol-resistant marker, and the transformation of the plasmid into the strain can be achieved by screening with chloramphenicol.

The recombinant vector pXMJ19-BBD29_09525 is a recombinant vector obtained by substituting a fragment (a small fragment) between *EcoR* I and *Kpn* I recognition sites in pXMJ19 vector with the DNA fragment set forth as positions 37-1927 of SEQ ID NO: 32 in the Sequence Listing, while keeping the other sequences of the pXMJ19 vector unchanged.

The recombinant vector pXMJ19-BBD29_09525^{C337T} is a recombinant vector obtained by substituting a fragment (a small fragment) between *EcoR* I and *Kpn* I recognition sites in pXMJ19 vector with the DNA fragment set forth as positions 37-1927 of SEQ ID NO: 33 in the Sequence Listing, while keeping the other sequences of the pXMJ19 vector unchanged.

PCR System: 10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL in a total volume of 50 µL.

The PCR amplification is performed by pre-denaturation for 5min at 94°C, denaturation for 30s at 94°C, annealing for 30s at 52°C, and extension for 120s at 72°C (30 circles), and overextension for 10min at 72°C.

The two plasmids (pXMJ19-BBD29_09525 and pXMJ19-BBD29_09525^{C337T}) are each introduced into the strain *Corynebacterium glutamicum* CGMCC No. 21220 by virtue of electrotransformation, and PCR identification is performed on the cultured mono-colonies with primers M13R (-48) and P18 for a transformed strain containing a fragment in about 2000 bp from PCR amplification, designated as YPG-010 (without a mutation point) and YPG-011 (with a mutation point).

The recombinant bacterium YPG-010 contains a double-copy BBD29_09525 gene as set forth in SEQ ID NO: 1; the recombinant bacterium YPG-010 is an engineered bacterium overexpressing a wild-type BBD29_09525 gene on a plasmid, i.e., overexpression outside a chromosome from plasmid pXMJ19-BBD29_09525.

The recombinant bacterium YPG-011 contains a mutated BBD29_09525^{C337T} gene as set forth in SEQ ID NO: 2; the recombinant bacterium YPG-011 is an engineered bacterium overexpressing a mutant BBD29_09525^{C337T} gene on a plasmid, i.e., overexpression outside a chromosome from plasmid pXMJ19-BBD29_09525^{C337T}.

### Example 5. Construction of Engineered Strain with BBD29_09525 Gene Deleted on Genome

According to the genome sequence of *Corynebacterium glutamicum* ATCC13869 published on NCBI, two pairs of primers for amplifying fragments at both ends of a BBD29_09525 gene coding region are synthesized, as upstream and downstream homologous arm fragments. The primers are designed as follows (synthesized by Invitrogen Corporation, Shanghai):

| | |
|---|---|
| P19: | 5' CAGTGCCAAGCTTGCATGCCTGCAGGTCGACTCTAGGCCTGACTGA TTTTGGGCTG 3' (SEQ ID NO: 23) |
| P20: | 5' GAGATAAAAGGAAGTTGAACATCTTCTAACTGCTTTCTTT 3' (SEQ ID NO: 24) |
| P21: | 5' AAAGAAAGCAGTTAGAAGATGTTCAACTTCCTTTTATCTC 3' (SEQ ID NO: 25) |
| P22: | 5'CAGCTATGACCATGATTACGAATTCGAGCTCGGTACCCAGATGACCTC GGCAGCAGCT 3' (SEQ ID NO: 26) |

Method for Construction: Using *Corynebacterium glutamicum* ATCC13869 as a template, PCR amplification is performed with primers P19/P20 and P21/P22, respectively, to provide an upstream homologous arm fragment in 804 bp and a downstream homologous arm fragment in 807 bp. Further, P19/P22 are used as primers for an overlap PCR to provide an integral homologous arm fragment in 1571 bp. After the PCR reaction is completed, electrophoresis is performed on the amplified products for recovering a desired DNA fragment in 1571 bp using a Column DNA Gel Recovery Kit, which is ligated, by virtue of NEBuider recombination system, to a shuttle plasmid, plasmid pk18mobsacB, having been cleaved with *Xba* I and recovered to provide a knockout plasmid. The plasmid contains a kanamycin-resistant marker.

The knockout plasmid is electrotransformed into the strain *Corynebacterium glutamicum* CGMCC No. 21220, and PCR identification is performed on the cultured mono-colonies each with the following primers (synthesized by Invitrogen Corporation, Shanghai):
P23: 5' GCCTGACTGA TTTTGGGCTG 3' (SEQ ID NO: 27)
P24: 5' AGATGACCTC GGCAGCAGCT 3' (SEQ ID NO: 28)

A strain with bands in 1497bp and 3000bp amplified from the above PCR amplification is a positive one, while a strain with just a band in 3000bp amplified therefrom is an original one. Having being screened on a culture medium with 15% sucrose, the positive strain is cultured on culture media with and without kanamycin, respectively, and strains that grow on a culture medium without kanamycin and do not grow on a culture medium with kanamycin are selected for further PCR identification with primers P23/P24. The strain with a band in 1497 bp amplified therefrom is a genetically engineered strain with the BBD29_09525 gene coding region knocked out, designated as YPG-012 (BBD29_09525 gene on the genome of *Corynebacterium glutamicum* CGMCC No. 21220 is knocked out).

### Example 6. Experiments on Fermentation of L-glutamic acid

Experiments on fermentation are performed on the strains (YPG-007, YPG-008, YPG-009, YPG-010, YPG-011 and YPG-012) constructed in the above Examples 2-5 and an original strain *Corynebacterium glutamicum* CGMCC No. 21220 in a fermentation tank, BLBIO-5GC-4-H type (purchased from Shanghai Bailun Biological Technology Co., Ltd.) with the culture medium shown in Table 3 and the control process shown in Table 4. Triplicates are done for each strain. Results are shown in Table 5.

**Table 3 Formulation of Culture Medium for Fermentation (with water as the rest)**

| Name of Reagent | Compounding Ratio |
|---|---|
| Glucose | 5.0 g/L |
| Phosphoric Acid | 0.38 g/L |
| Magnesium Sulfate | 1.85 g/L |
| Potassium Chloride | 1.6 g/L |
| Biotin | 550 µg/L |
| Vitamin B1 | 300 µg/L |
| Ferrous Sulfate | 10 mg/L |
| Manganese Sulfate | 10 g/dL |
| KH₂PO₄ | 2.8 g/L |
| Vitamin C | 0.75 mg/L |
| Vitamin B12 | 2.5 µg/L |
| Para-Aminobenzoic acid | 0.75 mg/L |
| Defoamer | 0.0015 mL/dL |
| Betaine | 1.5 g/L |
| Cane-Sugar Molasses | 7 mL/L |
| Corn Steep Liquor | 77 mL/L |
| Aspartic acid | 1.7 g/L |
| Hair powder | 2 g/L |

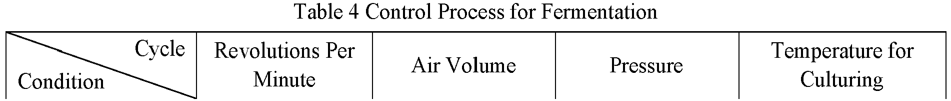

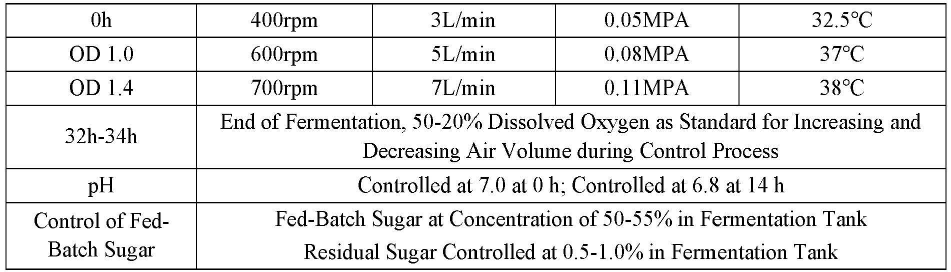

**Table 5 Results of Experiments on Fermentation of L-glutamic Acid**

| Strain | Production of L-Glutamic Acid (g/L) | OD (562nm) |
|---|---|---|
| *Corynebacterium glutamicum* CGMCC No. 21220 | 103.3 | 47.2 |
| YPG-007 | 102.6 | 45.3 |
| YPG-008 | 104.1 | 46.5 |
| YPG-009 | 105.3 | 46.9 |
| YPG-010 | 104.8 | 45.7 |
| YPG-011 | 106.1 | 45.3 |
| YPG-012 | 97.3 | 46.1 |

Results from Table 5 show that overexpression of the BBD29_09525 gene, or point mutation to the BBD29_09525 gene coding region, BBD29_09525^{C337T}, and/or overexpression of the BBD29_09525 gene or a mutant gene BBD29_09525^{C337T} thereof in *Corynebacterium glutamicum* facilitate an increase in the production of L-glutamic acid, while weakening or knocking out the BBD29_09525 gene is adverse to the accumulation of L-glutamic acid.

The present invention has been described in detail above. For those skilled in the art, the present invention can be implemented in a wide range with equivalent parameters, concentrations and conditions without departing from the spirit and scope of the present invention and without unnecessary experiments. Although specific examples have been provided in the present invention, it should be understood that further improvements can be made to the present invention. In summary, according to the principles of the present invention, the present application is intended to include any variations, uses, or improvements to the present invention, including changes made with routine techniques as known in the art, which depart from the scope disclosed in the present application. Some essential features can be applied in accordance with the scope of the following appended claims.

### Industrial Application

The present invention has found out that, by weakening or knocking out the BBD29_09525 gene, the product coded by the gene has an effect on L-glutamic acid production capacity, and that a recombinant strain obtained by introducing a point mutation to the coding sequence, or by increasing the copy number of, or overexpressing the gene facilitates production of glutamic acid at a higher concentration as compared with an unmodified strain.

Specifically, the present invention first constructs a genetically engineered bacterium YPG-007 with a point mutation (C-T) by introducing a point mutation to a BBD29_09525 gene coding region (SEQ ID NO: 1) of *Corynebacterium glutamicum* CGMCC No. 21220 via allelic replacement. In order to further investigate and verify that overexpressing a wild-type BBD29_09525 gene or a mutant gene BBD29_09525^{C337T} thereof in a producer bacterium can increase the production of L-glutamic acid, an exogenous gene is integrated into the chromosome of a host, or expressed outside the chromosome by a plasmid, respectively, thereby constructing engineered bacteria YPG-008, YPG-009, YPG-010 and YPG-011 overexpressing BBD29_09525 gene or BBD29_09525^{C337T} gene on genome and plasmid. Experiments suggest that BBD29_09525 gene and variants thereof are involved in the biosynthesis of L-glutamic acid. By overexpressing or knocking out BBD29_11265 gene, or having site-directed mutation (such as point mutation) thereto, the amount of accumulation of L-glutamic acid in a microorganism can be regulated. Point mutation to a BBD29_09525 gene coding region or overexpression of BBD29_09525 gene or a mutant gene BBD29_09525^{C337T} thereof in a producer bacterium facilitates an increase in the production and conversion rate of L-glutamic acid, while knocking out or weakening the BBD29_09525 gene is adverse to the accumulation of L-glutamic acid. BBD29_09525 gene and a variant thereof (such as BBD29_09525^{C337T} gene) can be used to construct a genetically engineered strain for producing L-glutamic acid to promote an increase in the production of L-glutamic acid, and to breed a high-production and high-quality strain for industrialized production, which finds values in a wide range of applications to, and is of important economic significance for industrialized production of L-glutamic acid.

## Claims

1. A bacterium for generating L-glutamic acid, **characterized in** having an improved expression of a polynucleotide encoding an amino acid sequence of SEQ ID NO: 3 or a homologous sequence thereof;
preferably, the improved expression is an enhanced expression of the polynucleotide encoding an amino acid sequence of SEQ ID NO: 3 or a homologous sequence thereof, or having a point mutation in the polynucleotide encoding an amino acid sequence of SEQ ID NO: 3 or a homologous sequence thereof, or having a point mutation in, and an enhanced expression of the polynucleotide encoding an amino acid sequence of SEQ ID NO: 3 or a homologous sequence thereof.

2. The bacterium of claim 1, **characterized in** a point mutation to the polynucleotide encoding an amino acid sequence of SEQ ID NO: 3 such that proline at position 113 in the amino acid sequence of SEQ ID NO: 3 is substituted with a different amino acid; preferably, proline at position 113 is substituted with serine.

3. The bacterium of any one of claims 1-2, **characterized in that** the polynucleotide encoding an amino acid sequence of SEQ ID NO: 3 comprises a nucleotide sequence of SEQ ID NO: 1.

4. The bacterium of any one of claims 1-3, **characterized in that** the polynucleotide sequence having a point mutation is formed from a mutation to the base at position 337 of a polynucleotide sequence set forth in SEQ ID NO: 1;
preferably, the mutation comprises a mutation of the base at position 337 of a polynucleotide sequence set forth in SEQ ID NO: 1 from cytosine (C) to thymine (T);
preferably, the polynucleotide sequence having a point mutation comprises a polynucleotide sequence set forth in SEQ ID NO: 2.

5. The bacterium of any one of claims 1-4, **characterized in that** the bacterium is a bacterium of the genus *Corynebacterium,* preferably, *Corynebacterium acetoacidophilum*, *Corynebacterium acetoglutamicum*, *Corynebacterium callunae*, *Corynebacterium glutamicum, Brevibacterium flavum, Brevibacterium lactofermentum*, *Corynebacterium ammoniagenes, Corynebacterium pekinense, Brevibacterium saccharolyticum, Brevibacterium roseum,* and *Brevibacterium thiogenitalis*; more preferably, *Corynebacterium glutamicum* CGMCC No. 21220 or ATCC 13869.

6. A polynucleotide sequence, **characterized in** comprising a polynucleotide encoding containing an amino acid sequence set forth in SEQ ID NO: 3, wherein proline at position 113 is substituted with a different amino acid; preferably, proline at position 113 is substituted with serine;
preferably, the polynucleotide sequence comprises a polynucleotide encoding containing an amino acid sequence set forth in SEQ ID NO: 4;
preferably, the polynucleotide sequence is formed from a mutation to the base at position 337 of a polynucleotide sequence set forth in SEQ ID NO: 1; preferably, the mutation is a mutation of the base at position 337 of the polynucleotide sequence set forth in SEQ ID NO: 1 from cytosine (C) to thymine (T);
preferably, the polynucleotide sequence comprises a polynucleotide sequence set forth in SEQ ID NO: 2.

7. An amino acid sequence, **characterized in that** the sequence is set forth in SEQ ID NO: 4.

8. A recombinant vector, **characterized in** comprising the polynucleotide sequence of claim 6.

9. A recombinant strain, **characterized in** comprising the polynucleotide sequence of claim 6.

10. A method for producing L-glutamic acid, the method comprising: culturing the bacterium of any one of claims 1-5 and recovering L-glutamic acid from the culture.

11. A protein, **characterized in that** the protein is any one of:
A1) a protein whose amino acid sequence is SEQ ID NO: 4;
A2) a protein having 80% or more identity to and the same function as the protein indicated in A1), as obtained by subjecting the amino acid sequence set forth in SEQ ID NO: 4 to substitution and/or deletion and/or addition of amino acid residues;
A3) a fusion protein having the same function, as obtained by linking a tag to the N-terminus and/or C-terminus of A1) or A2).

12. A nucleic acid molecule, **characterized in that** the nucleic acid molecule is any one of:
B1) a nucleic acid molecule encoding the protein of claim 11;
B2) a DNA molecule whose coding sequence is set forth in SEQ ID NO: 2;
B3) a DNA molecule whose nucleotide sequence is set forth in SEQ ID NO: 2.

13. A biomaterial, **characterized in that** the biomaterial is any one of:
C1) an expression cassette comprising the nucleic acid molecule of claim 12;
C2) a recombinant vector comprising the nucleic acid molecule of claim 12, or a recombinant vector comprising the expression cassette of C1);
C3) a recombinant microorganism comprising the nucleic acid molecule of claim 12, or a recombinant microorganism comprising the expression cassette of C1), or a recombinant microorganism comprising the recombinant vector of C2).

14. Any one of the following uses of any one of D1)-D8):
F1) use of any one of D1)-D8) in the regulation of the production of L-glutamic acid of a microorganism;
F2) use of any one of D1)-D8) in the construction of a genetically engineered bacterium for producing L-glutamic acid;
F3) use of any one of D1)-D8) in the preparation of L-glutamic acid;
wherein the D1)-D8) is:
D1) the protein of claim 11;
D2) the nucleic acid molecule of claim 12;
D3) the biomaterial of claim 13;
D4) a DNA molecule whose nucleotide sequence is SEQ ID NO: 1;
D5) a DNA molecule having 90% or more identity to and the same function as the DNA molecule set forth in SEQ ID NO: 1, as obtained by subjecting the nucleotide sequence set forth in SEQ ID NO: 1 to modification, and/or substitution and/or deletion and/or addition of one or several nucleotides;
D6) an expression cassette comprising the DNA molecule in D4) or D5);
D7) a recombinant vector comprising the DNA molecule in D4) or D5), or a recombinant vector comprising the expression cassette of D6);
D8) a recombinant microorganism comprising the DNA molecule in D4) or D5), or a recombinant microorganism comprising the expression cassette of D6), or a recombinant microorganism comprising the recombinant vector of D7).

15. A method for increasing the production of L-glutamic acid in a microorganism, **characterized in that** the method comprises any one of:
E1) increasing the expression amount, or content of the nucleic acid molecule of claim 12 in a target microorganism to provide a microorganism having a greater production of L-glutamic acid than the target microorganism;
E2) increasing the expression amount, or content of the DNA molecule of D4) or D5) in claim 14 in a target microorganism to provide a microorganism having a greater production of L-glutamic acid than the target microorganism;
E3) performing a mutation on the DNA molecule whose nucleotide sequence is SEQ ID NO: 1 in the target microorganism to provide a microorganism having a greater production of L-glutamic acid than the target microorganism.

16. The method of claim 15, **characterized in that** the mutation is a point mutation.

17. The method of claim 16, **characterized in that** the point mutation is a mutation of proline residue at position 113 in an amino acid sequence coded by the DNA molecule set forth in SEQ ID NO: 1 to another amino acid residue.

18. The method of claim 16 or 17, **characterized in that** the point mutation is a mutation of proline at position 113 in an amino acid sequence coded by the DNA molecule set forth in SEQ ID NO: 1 to serine, providing a mutated protein whose amino acid sequence is SEQ ID NO: 4.

19. A method for constructing the recombinant microorganism of claim 13 or 14, **characterized in that** the method comprises at least one of:
F1) introducing the nucleic acid molecule of claim 12 into a target microorganism to provide the recombinant microorganism;
F2) introducing the DNA molecule set forth in SEQ ID NO: 1 into a target microorganism to provide the recombinant microorganism;
F3) editing the DNA molecule set forth in SEQ ID NO: 1 with a gene editing measure to contain the DNA molecule set forth in SEQ ID NO: 2 in a target microorganism.

20. A method for preparing L-glutamic acid, **characterized in that** the method comprises producing L-glutamic acid with the recombinant microorganism of claim 13 or 14.
